# EUROPEAN PATENT APPLICATION

(11) **EP 4 166 064 A1**
(43) Date of publication of application: **19.04.2023**
(21) Application number: 21825524.8
(22) Date of filing: 27.04.2021
(51) Int. Cl.: A61B 3/024

(54) **VIEWING FIELD TEST DEVICE**

(30) Priority: 16.06.2020 JP 2020103472
(71) Applicant: FINDEX Inc., Chiyoda-ku, Tokyo 100-0004 (JP)
(72) Inventor: AIBARA Teruo, Matsuyama-shi, Ehime 790-0003 (JP)
(74) Representative: Zimmermann & Partner Patentanwälte mbB
(86) International application number: PCT/JP2021/016860
(87) International publication number: WO 2021/256100

(57) **Abstract**

PROBLEM: To provide a visual field testing apparatus such as will permit visual field testing to be carried out even on a user who has a scotoma in the vicinity of the center of the visual field.

SOLUTION MEANS: Visual field testing apparatus 1 comprises display 13; line of sight detection unit 31 that detects a line of sight of a user and outputs line of sight information pertaining to a direction of the line of sight; visual recognition determination unit 40 that carries out determination as to whether or not the user has visually recognized the target based on the line of sight information and position information of the target; a target display unit 33 that causes the target in the form of a set consisting of a gazing target and a measurement target to be sequentially displayed at display 13; and visual field testing unit 50 that carries out visual field testing by causing visual recognition determination to be carried out with respect to the measurement target by visual recognition determination unit 40 as the gazing target and the measurement target are sequentially displayed by target display unit 33; wherein display 13 has display 13a for the right eye and display 13b for the left eye; and wherein visual field testing unit 50 has a central scotoma possessor testing mode in which the gazing target is displayed at the display 13 for the opposite eye.

## Description

### TECHNICAL FIELD

The present invention relates to a visual field testing apparatus.

### BACKGROUND ART

Visual field testing apparatuses for testing visual field have been provided conventionally; for example, there is such a disclosure at Patent Reference No. 1, below. At the visual field testing apparatus disclosed at Patent Reference No. 1, below, a user is made to use a button or other such input apparatus to input whether or not a target has been seen by his or her eye and recognized.

However, depending on the user, there have been cases in which the user is unfamiliar with operation of the input apparatus, or due to tension or the like is unable to skillfully operate the input apparatus, and so fails to properly carry out input therewith, as a result of which there has been inability to accurately carry out visual field testing.

In contradistinction thereto, a visual field testing apparatus is disclosed at Patent Reference No. 2, below, in which the line of sight which is the direction in which a user is looking is automatically detected by a line of sight detection apparatus, and determination is automatically made as to whether or not the user visually recognizes the target.

### PRIOR ART REFERENCES

### PATENT REFERENCES

Patent Reference No. 1: WO 2017-022757
Patent Reference No. 2: Japanese Patent Application Publication Kokai No. 2011-161122

### SUMMARY OF INVENTION

### PROBLEM TO BE SOLVED BY INVENTION

Here, during ordinary visual field testing, two targets-these being a gazing target for causing the line of sight to return to the center where gazing (ocular fixation) is made to take place, and a measurement target for measuring visual field-are alternately used. Accordingly, there is occurrence of situations in which a user who has a scotoma in the vicinity of the center of the visual field is unable to visually recognize the gazing target and is unable to carry out visual field testing.

The present invention was conceived in light of such problems, it being an object thereof to provide a visual field testing apparatus such as will permit visual field testing to be carried out even on a user who has a scotoma in the vicinity of the center of the visual field.

### MEANS FOR SOLVING PROBLEM

To solve the foregoing problems, in the context of a visual field testing apparatus for testing a range of visual field of a user, a visual field testing apparatus associated with the present invention is characterized in that it comprises a display for displaying a target; a line of sight detection unit that detects a line of sight of the user and that outputs line of sight information pertaining to a direction of the line of sight; a visual recognition determination unit that carries out determination as to whether or not the user has visually recognized the target based on the line of sight information and position information of the target; a target display unit that causes the target in a form of a set consisting of a gazing target and a measurement target to be sequentially displayed at the display; a visual field testing unit that carries out visual field testing by causing visual recognition determination to be carried out with respect to the measurement target by the target visual recognition determination unit as the gazing target and the measurement target are sequentially displayed by the target display unit; wherein the display has a display for a right eye and a display for a left eye; and wherein the visual field testing unit has a central scotoma possessor testing mode in which the target display unit is controlled so as to cause the gazing target to be displayed by whichever of the display for the right eye or the display for the left eye is opposite an eye being tested.

### BENEFIT OF INVENTION

In accordance with the present invention, a visual field testing unit may have a central scotoma possessor mode, and visual field testing may be properly carried out even on a user who has a scotoma in the vicinity of the center of the visual field.

### BRIEF DESCRIPTION OF DRAWINGS

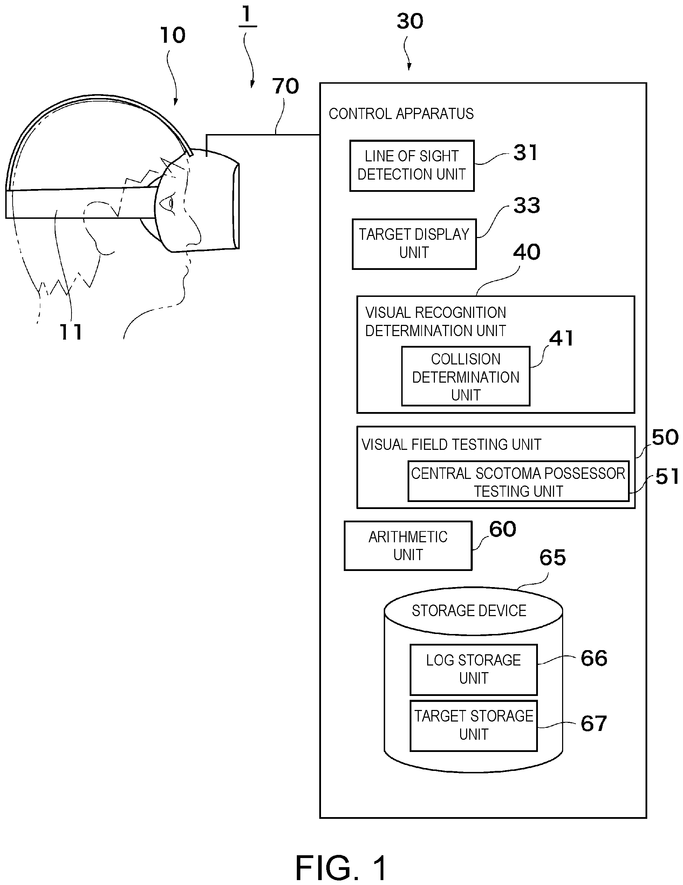

[FIG. 1] FIG. 1 is a schematic diagram showing in simplified fashion the constitution of a visual field testing apparatus associated with an embodiment of the present invention.

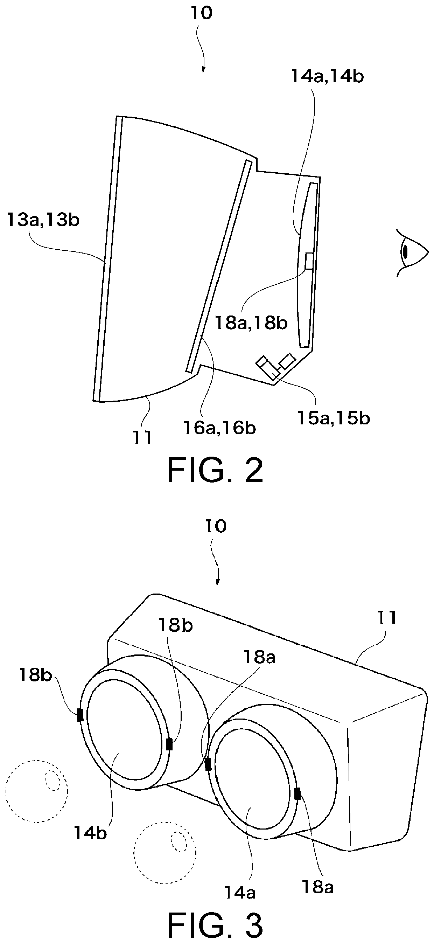

[FIG. 2] FIG. 2 is a sectional view of the principal components in, and showing in simplified fashion the constitution of, an HMD associated with an embodiment of the present invention.
[FIG. 3] FIG. 3 is a perspective view of the principal components in, and showing in simplified fashion the constitution of, an HMD associated with an embodiment of the present invention.
[FIG. 4] FIG. 4 is a drawing showing locations at which measurement targets associated with an embodiment of the present invention might be displayed.
[FIG. 5] FIG. 5 is a drawing showing examples of output screens displaying results of visual field testing associated with an embodiment of the present invention.

### EMBODIMENTS FOR CARRYING OUT INVENTION

Visual field testing apparatus 1, which is an embodiment of the present invention, will be described in detail hereinbelow with reference to the drawings. In accordance with the present embodiment, visual field testing apparatus 1 carries out visual field testing while automatically detecting a line of sight which is a direction in which the user is looking with his or her eye(s) and causing a determination to be made as to whether a target which is a visual symbol that is displayed at a display apparatus been seen by the eye(s) of the user and recognized, i.e., whether or not it has been visually recognized.

Visual field testing apparatus 1 comprises head mounted display (HMD) 10, control apparatus 30, and communication cable 70 which connects HMD 10 and control apparatus 30.

HMD 10 comprises case 11 which includes a belt for mounting thereof on the head of a user who is a test subject, display 13, convex lens 14, camera 15, hot mirror 16, and near-infrared emitter 18, and is equipped with line of sight detection functionality for cooperating with line of sight detection unit 31, described below, to automatically detect a line of sight which is a direction in which the user on whom HMD 10 is mounted is looking with his or her eye(s).

Display 13 is a liquid crystal display in which display 13a for the right eye of the user and display 13b for the left eye thereof are installed so as to respectively face the fronts of his or her left and right eyes. Respectively installed to the right and left between display 13 and the eyes of the user are convex lens 14a for the right eye and convex lens 14b for the left eye. An image displayed at display 13 will appear at the eye(s) of the user by way of convex lens 14.

Camera 15 is a near-infrared camera for capturing images of the eyes of the user, video capture of the left and right eyes of the user being carried out thereby through use of near-infrared light which is nonvisible light. Camera 15a for the right eye and camera 15b for the left eye are likewise installed at camera 15.

Respectively installed to the right and left between display 13 and convex lens 14 are hot mirrors 16a, 16b having multilayer films which reflect near-infrared light and allow visible light to pass therethrough. Visible light of an image irradiated from display 13 passes through hot mirror 16, and nonvisible light of near-infrared light irradiated from emitter 18 is reflected by hot mirror 16.

Emitter 18 is an LED (IR-LED) that causes near-infrared light to be irradiated as illumination for capturing images of the eyes of the user. Emitters 18a, 18b are respectively installed to the left and right so as to face the left and right eyes of the user at the periphery of convex lens 14.

Camera 15 is installed at the side toward the eyes, which is the side opposite display 13, from hot mirror 16. Near-infrared light which directly irradiates the eyes of the user from emitter 18 is reflected by the eyes of the user and thereafter passes through convex lens 14 and is reflected by hot mirror 16 to arrive at camera 15 where an image is captured therefrom.

Control apparatus 30 comprises central processing unit(s) (CPU) or other such arithmetic unit(s) 60 for carrying out various types of operations, as well as hard disc drive(s) (HDD) for storing various types of information, and/or random access memory or memories (RAM) capable of being used as a work area during arithmetic processing, or other such storage device(s) 65.

Storage device 65 comprises line of sight log storage unit 66 which records line of sight information detected by line of sight detection unit 31, described below; and target storage unit 67 which records target information to be displayed at display 13.

Furthermore, control apparatus 30 comprises-in terms of functionalities-line of sight detection unit 31, target display unit 33, visual recognition determination unit 40, and visual field testing unit 50, these functionalities being implemented by causing prescribed program(s) stored at storage device 65 to be executed by arithmetic unit 60.

Line of sight detection unit 31 detects the direction in which the user is looking, i.e., the line of sight, based on captured image(s) of the eye(s) of the user which is the output of camera 15. More specifically, line of sight detection unit 31 outputs line of sight information indicating the direction of the line of sight in the form of two angular components (θ, φ) which are polar coordinates (spherical coordinates) as line of sight information (line of sight angle).

Establishment of the polar coordinate system may be carried out during the calibration which takes place prior to start of visual field measurement. More specifically, the user might be made to stare at a previously designated target and the image thereof might be made to correspond to the polar coordinate system origin (center of eyeball), following which an image at which the user is made to stare at a point representing a different designated set of polar coordinates might be stored. By determining the correlation between the locations of the centers of the pupils captured in the respective images and the respective polar coordinates at which the user was made to stare, it is possible to determine the line of sight angle of the polar coordinates from the images.

This line of sight detection is carried out independently for the right eye and the left eye, line of sight information being recorded as a function of time at line of sight log storage unit 66 at intervals of approximately 15 ms. Included within the line of sight information which is recorded at line of sight log storage unit 66 are the aforementioned two angular components (θ, φ) which are polar coordinates (spherical coordinates).

Target display unit 33 causes targets of prescribed size(s) to be sequentially displayed at prescribed locations(s) at display 13 based on target information recorded at target storage unit 67. Here, in accordance with the present embodiment, targets in the form of two targets-these being a gazing target for causing the line of sight to return to a location in the vicinity of the center where gazing (ocular fixation) is made to take place, and a measurement target for measuring visual field-are used, a target set which consists of a measurement target and a gazing target being recorded at target storage unit 67.

Note that location information indicating display locations of respective targets in the form of sets of the aforesaid two angular components (θ, φ) which are polar coordinates may be used as the target information which is stored at target storage unit 67. These two angular components define a target angle indicating a target location expressed in angular form.

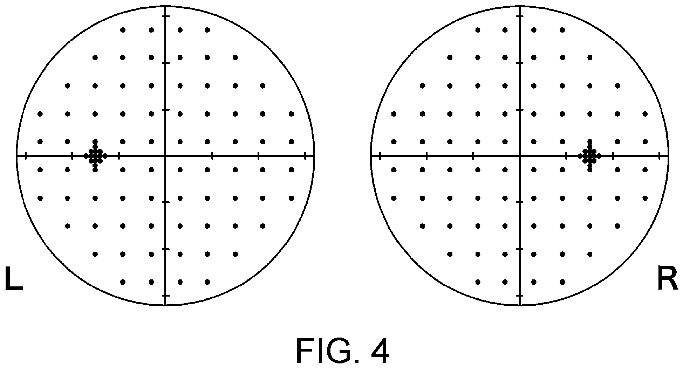

FIG. 4 shows display locations of measurement targets. In accordance with the present embodiment, to carry out measurement of visual field within a visual field angle of 30°, measurement targets are displayed at points at 76 locations arranged after the fashion of the intersections of a grid, and at points at 13 locations arranged in concentrated fashion in the vicinity of the blind spot, for a total of 89 locations (Humphrey visual field test; center 30-2). The visual field testing method may of course be varied as appropriate, and the locations at which the targets are displayed at display 13 and so forth may be varied as appropriate in correspondence to the visual field testing method employed.

Note that in accordance with the present embodiment not all measurement targets are displayed at the coordinate locations shown in FIG. 4, display locations being adjusted as appropriate so as to cause all measurement targets to be displayed within a prescribed visual field angle (18° in the present embodiment) at display 13. Such adjustment may be carried out in advance prior to measurement, the post-adjustment locations being recorded in the form of target information at target storage unit 67. Furthermore, the gazing target is not stationary with respect to the center of display 13 but is displayed at a location which has been made to agree with the adjustment of the measurement target (the location for which the visual field angle of the post-adjustment measurement target is the same as was the case prior to adjustment).

The reason for this is, because measurement targets are displayed at locations which are removed by some distances from the center of display 13, and because the larger the line of sight angle of the line of sight with respect to the screen at display 13 the lower will be the precision with which the line of sight will be detected by line of sight detection unit 31, to cause the measurement targets to be displayed so as to be located within a prescribed visual field angle (18° in the present embodiment) from the center of the optical system at display 13.

More specifically, if a measurement target would be at a location outside the range of a visual field angle of 18° from the center of the optical system at display 13, adjustment is carried out so as to cause said measurement target to be slidingly moved to a location where it will be displayed within 18° at display 13. At such time, so that there is no change in the angle by which the line of sight moves in going from the immediately prior gazing target in that same set to said measurement target, the immediately prior gazing target is likewise made to slide by the same distance and in the same direction as said measurement target.

By thus causing measurement targets to be displayed at locations within a visual field angle of 18° at display 13, it is possible to detect the line of sight in stable and highly precise fashion without experiencing decrease in the precision of detection thereof by line of sight detection unit 31.

Visual recognition determination unit 40 which comprises collision determination unit 41 carries out determination as to whether or not a target has been visually recognized by a user based on a line of sight detected by line of sight detection unit 31 and the coordinates of a measurement target displayed at display 13.

Collision determination unit 41 carries out determination as to whether or not the target has been visually recognized by the user based on whether or not an imaginary line that is an extension of a line of sight vector direction detected by line of sight detection unit 31 within a prescribed coordinate system would physically collide with the target in question.

In accordance with present embodiment, a determination is made that there has been a collision and that visual recognition has taken place if upon comparison of the line of sight angle detected by line of sight detection unit 31 and the target angle indicating the location of the target in question, the angular difference therebetween is within 2° within the same polar coordinate system.

Visual field testing unit 50 controls target display unit 33 and visual recognition determination unit 40, and causes visual field testing of the user to be carried out by sequentially causing determination with respect to visual recognition of respective targets to be carried out by visual recognition determination unit 40 while causing sets of gazing targets and measurement targets to be sequentially displayed at prescribed locations by target display unit 33.

When in normal mode, to carry out visual field testing of the right eye, target display unit 33 causes targets to be displayed at only display 13a for the right eye; and to carry out visual field testing of the left eye, target display unit 33 causes targets to be displayed at only display 13b for the left eye.

Visual field testing unit 50 comprises central scotoma possessor testing unit 51. Central scotoma possessor testing unit 51 performs visual field testing in central scotoma possessor mode which is for carrying out visual field testing of a central scotoma possessing user who has a scotoma in the vicinity of the center of the left and/or right eye.

Because the gazing target for causing the line of sight to return to a location in the vicinity of the center is displayed in the vicinity of the center, it is sometimes the case that a central scotoma possessing user will be unable to visually recognize the gazing target, as a result of which it will be impossible to carry out testing through use of the visual field testing of the aforementioned normal mode.

To address this, when in central scotoma possessor mode, central scotoma possessor testing unit 51 causes the gazing target-which is part of the set of targets consisting of the gazing target and the measurement target-to be displayed each time at the display of display 13 which is for the eye opposite the eye being tested.

For example, when carrying out visual field testing of the right eye of a user who has a scotoma in the vicinity of the center of the right eye, control might be carried out by central scotoma possessor testing unit 51 so as to cause target display unit 33 to display all gazing targets at display 13b for the left eye, and display all measurement targets at display 13a for the right eye.

Based on the knowledge that the left and right eyes of a human being are ordinarily such that the lines of sight of the left and right eyes are approximately parallel, if the gazing target is displayed at the display of display 13 which is for the eye opposite the eye being tested, causing visual recognition of the gazing target to be carried out by this opposite eye will allow the line of sight of the eye being tested to also be made to return to the center. It is therefore possible for visual field testing of a central scotoma possessing user to be carried out not in normal mode but as a result of causing a central scotoma possessor mode to be provided by central scotoma possessor testing unit 51.

Moreover, in accordance with the present embodiment, when the gazing target is displayed at the display of display 13 which is for the opposite eye, central scotoma possessor testing unit 51 carries out control of target display unit 33 so as to cause brightness to be increased to a value greater than would normally be the case, and so as to cause the gazing target to be displayed larger than would normally be the case.

More specifically, whereas a normal target is displayed by display 13 as a circle 4 mm² in size and having a brightness of 413 cd/m², the gazing target when in central scotoma possessor testing mode is displayed so as to be 16 mm² in size and having a brightness of 826 cd/m², which is a brightness ratio of 2x, and an area ratio of 4x (radius ratio of 2x). Note that size of the target is expressed in terms of the size thereof when displayed on a screen which is 30 cm in front of the eye.

Because there is a possibility that a user who has a central scotoma at one eye will have decreased sensitivity at the opposite eye, by thus causing a gazing target which is brighter and larger than normal to be displayed at the opposite eye when in central scotoma possessor testing mode, it will be possible to more properly carry out visual field testing.

It is of course possible to adjust as appropriate the brightness and/or size of the gazing target which is displayed at the eye opposite the eye being tested, and while these may be made to be the same as in the normal situation, based upon consideration of the decrease in sensitivity of the eyes of central scotoma possessors, the reliability of testing, and so forth, it is preferred that the brightness and size of the gazing target should each be 1.5x to 3x that of the normal situation.

The foregoing being description of the constitution of visual field testing apparatus 1, description will next be given with regard to a visual field testing method that employs visual field testing apparatus 1. Visual field testing apparatus 1 performs visual field testing by executing visual field testing program(s) stored at storage device 65. Description is first given with respect to normal visual field testing which is carried out when in normal mode.

During visual field testing, HMD 10 is placed on the head of a user who is a test subject, and an instruction is given to the test subject to the effect that "targets will be sequentially displayed at display 13, so please keep looking at the targets". In carrying out measurement, calibration is first carried out, adjustment being carried out so that line of sight detection unit 31 can accurately detect the line of sight of the test subject.

Following initial adjustment, target display unit 33 causes targets to be displayed at display 13. Target display unit 33 causes targets for testing of the right eye and targets for testing of the left eye to both be displayed in random and sequential fashion at display 13 based on target information recorded at target storage unit 67. That is, in accordance with the present embodiment, visual field testing of the right eye and visual field testing of the left eye are carried out simultaneously.

Here, because targets for the left eye are displayed only at display 13b for the left eye, and targets for the right eye are displayed only at display 13a for the right eye, only a target for either the right eye or the left eye will be displayed at display 13a for the right eye or display 13b for the left eye at any given time during testing.

As described above, a gazing target and a measurement target are recorded as a target set, and target display unit 33, before causing display of a measurement target, causes display of the gazing target which is part of the same set as that measurement target, and thereafter causes display of that measurement target. That is, target display unit 33 causes gazing targets and measurement targets to be displayed at display 13 in alternating fashion.

Switching of targets is such that when a determination is made by visual recognition determination unit 40 that a target which is being displayed has been visually recognized, target display unit 33 causes switching to the next target to occur and causes this to be displayed. Furthermore, following display of a target, if visual recognition does not occur despite passage of 2.5 s, this is taken to be a timeout condition, a determination is made that visual recognition did not occur, and the next target set is displayed. Note that where a target that resulted in generation of a timeout condition is a measurement target, said measurement target is scheduled for remeasurement. Note, however, that depending on the conditions under which each of the various test patterns are terminated, there may be cases in which it is not remeasured.

As targets are sequentially displayed at display 13 by target display unit 33 in this fashion, the line of sight of the test subject is detected by line of sight detection unit 31, and visual recognition determination unit 40 determines whether or not recognition by the test subject has taken place for each target based on the line of sight information which is the output of line of sight detection unit 31.

Visual recognition determination unit 40 is such that gazing targets and measurement targets which are displayed in sequential and alternating fashion are subjected to visual recognition determination which is sequentially carried out by collision determination unit 41 based on line of sight information which is sequentially output from line of sight detection unit 31 following display of respective targets. In the event that a determination is made by collision determination unit 41 that a target has not been recognized and said target is a measurement target, visual recognition determination unit 40 causes said measurement target to be scheduled for remeasurement.

Note, however, that depending on the conditions under which each of the various test patterns are terminated, there may be cases in which it is not remeasured. When one pass of visual recognition determination has been carried out on all measurement targets, then this is followed by visual recognition determination which is again carried out by visual recognition determination unit 40 on any measurement target(s) that were scheduled for remeasurement.

Target sets scheduled for remeasurement are also displayed in random and sequential fashion at display 13, and visual recognition determination is carried out. Where a measurement target cannot be determined to have been visually recognized despite remeasurement thereof, a decision is rendered by visual field testing unit 50 that there is a scotoma thereat; and where a measurement target is determined to have been visually recognized upon remeasurement thereof, this is scheduled to be measured yet again. Where a measurement target is also determined to have been visually recognized the third time that it is measured, a decision is rendered by visual field testing unit 50 that there is no scotoma thereat; where a measurement target is determined not to have been visually recognized when measured for the third time, a decision is rendered by visual field testing unit 50 that there is a scotoma thereat.

Moreover, where a determination is made by collision determination unit 41 that visual recognition has occurred, the time from display of said measurement target until visual recognition thereof by the test subject, i.e., the time from display of the measurement target until detection of the line of sight that collided therewith, is recorded as the response time at storage device 65.

Where this response time is not greater than 100 ms, because this is too short a response time, and it is conceivable that the reason for this could be that a subsequent target just happened to have been acquired in the instant that the target was switched, or that where a previous target and a subsequent target are close this created a situation in which the line of sight and the target just happened to collide in the instant that the target was switched, collision determination unit 41 treats this as an invalid visual recognition determination, and said measurement target is scheduled for remeasurement.

Furthermore, in the event that the response time is a prescribed time or longer, collision determination unit 41 treats this as a situation in which too much time has been taken for response, and causes said measurement target to be scheduled for remeasurement. Here, it is only measurement targets that are scheduled for remeasurement.

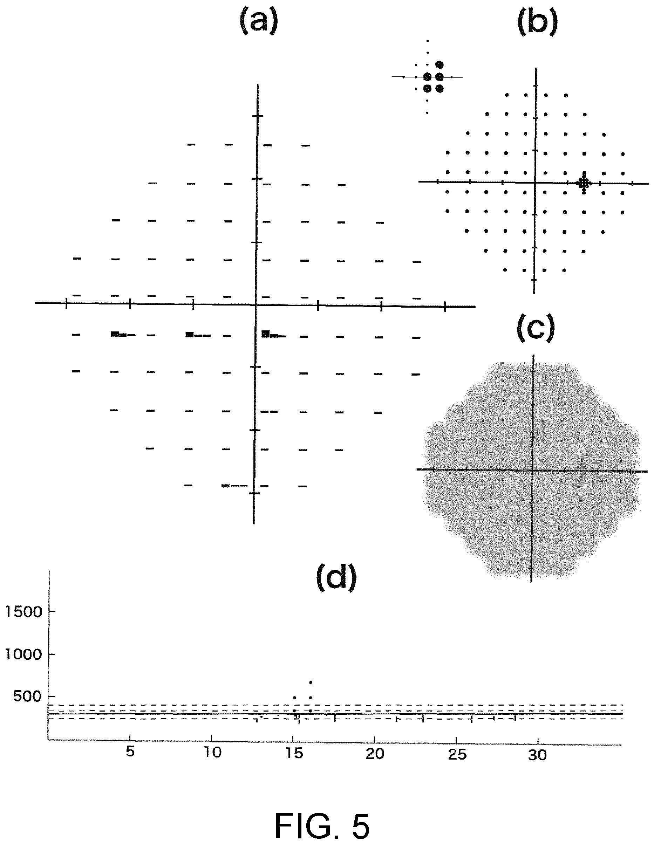

When visual recognition determination of all target sets-including those scheduled for remeasurement-as described above has been completed, visual field testing unit 50 outputs the results of visual field testing to a prescribed display apparatus (not shown) based on the results of visual recognition carried out by visual recognition determination unit 40. FIG. 5 shows examples of output screens displaying results of visual field testing.

At (a) in FIG. 5, heights indicate response times at respective measurement targets exclusive of the region of the blind spot, multiple measurement results for targets subject to remeasurement being shown in horizontally clustered fashion. At (b) in FIG. 5, locations of measurement targets at which decisions were rendered that there was a scotoma thereat are shown, measurement targets at which decisions were rendered that there was a scotoma thereat being indicated by large circles.

At (c) in FIG. 5, respective measurement targets together with both the response times thereat as well as the locations at which decisions were rendered that there was a scotoma thereat are indicated by color and density, regions in the vicinity of targets at which response time was short being shown in light green, the shade of green shown being darker the longer the response time thereat, with regions in the vicinity of scotomas being shown in red. At (d) in FIG. 5, measurement target response time is shown as a function of visual field angle, the horizontal axis being visual field angle, and the vertical axis being response time.

The foregoing being description with respect to visual field testing when in normal mode, description will next be given with respect to a method for visual field testing when in central scotoma possessor mode such as may be carried out by central scotoma possessor testing unit 51, description being primarily given with respect to the portions thereof that are different from normal mode. When in central scotoma possessor mode which is for visual field testing of a user who has a central scotoma at one eye, the method for displaying gazing targets is different from that employed in the foregoing normal mode.

Following initial adjustment, in causing targets to be displayed at display 13 by target display unit 33, during testing of the eye at which the user has a central scotoma, central scotoma possessor testing unit 51 of visual field testing unit 50 controls target display unit 33 so as to cause all gazing targets to be displayed at the display of display 13 which is for the eye opposite the eye being tested.

As a result, because in carrying out visual field testing, for example, of the right eye of a user who has a central scotoma at the right eye, all gazing targets would be displayed at display 13b for the left eye, by using the left eye to visually recognize the gazing target it will be possible to cause the line of sight of the right eye to be made to return to the vicinity of the center, and it will be possible to properly carry out visual field testing using measurement targets displayed at display 13a for the right eye. Of course, in carrying out visual field testing of the left eye at which there is no central scotoma, the gazing targets and the measurement targets would both be displayed at display 13b for the left eye in similar fashion as when in normal mode.

As targets are sequentially displayed at display 13 by target display unit 33 in this fashion, the line of sight of the test subject is detected by line of sight detection unit 31, and visual recognition determination unit 40 determines whether or not recognition by the test subject has taken place for each target based on the line of sight information which is the output of line of sight detection unit 31, as a result of which visual field testing unit 50 causes the results of visual field testing to be output based on the results of visual recognition carried out by visual recognition determination unit 40 in similar fashion as when in the foregoing normal mode.

Above, description has been given with respect to visual field testing apparatus 1 associated with the present embodiment; in accordance with the present embodiment, visual field testing unit 50 comprises central scotoma possessor testing unit 51, and by causing operations to be carried out in central scotoma possessor mode it is possible to properly carry out visual field testing of a user who has a central scotoma at only one eye.

While embodiments of the present invention have been described above, embodiments for carrying out the present invention are not limited to the foregoing embodiments, a great many variations being possible without departing from the gist of the present invention. For example, whereas the foregoing embodiment employed a noncontact-type line of sight detection unit in which the point on which the eye is fixated is estimated from the pattern of near-infrared light that is reflected from the cornea, so long as it permits detection of the line of sight of he user, it is possible to employ a contact-type line of sight detection unit or other such appropriate type of line of sight detection unit.

Furthermore, whereas in the foregoing embodiment the visual recognition determination unit carried out determination as to whether or not the user had visually recognized the target based on a determination made by a collision determination unit as to whether or not an imaginary line that is an extension of the direction of a line of sight detected by a line of sight detection unit would physically collide with the target in question, it is also possible to cause visual recognition determination to be carried out using logical determinative techniques that make use of logged line of sight information.

Furthermore, whereas the foregoing embodiment employed a polar coordinate system for lines of sight and line of sight position information, it is also possible to employ a three-dimensional rectangular coordinate system therefor. Furthermore, whereas in the foregoing embodiment independent displays were used for the display for the left eye and the display for the right eye, it is also possible to use a single large display that has been divided into a region for the right eye and a region for the left eye.

### EXPLANATION OF REFERENCE NUMERALS

- 1: Visual field testing apparatus
- 10: HMD
- 11: Case
- 13: Display
- 14: Convex lens
- 15: Camera
- 16: Hot mirror
- 18: Emitter
- 30: Control apparatus
- 31: Line of sight detection unit
- 33: Target display unit
- 40: Visual recognition determination unit
- 41: Collision determination unit
- 50: Visual field testing unit
- 51: Central scotoma possessor testing unit
- 60: Arithmetic unit
- 65: Storage device
- 66: Line of sight log storage unit
- 67: Target storage unit
- 70: Communication cable

## Claims

1. In the context of a visual field testing apparatus for testing a range of visual field of a user, a visual field testing apparatus **characterized in that** it comprises:
a display for displaying a target;
a line of sight detection unit that detects a line of sight of the user and that outputs line of sight information pertaining to a direction of the line of sight;
a visual recognition determination unit that carries out determination as to whether or not the user has visually recognized the target based on the line of sight information and position information of the target;
a target display unit that causes the target in a form of a set consisting of a gazing target and a measurement target to be sequentially displayed at the display; and
a visual field testing unit that carries out visual field testing by causing visual recognition determination to be carried out with respect to the measurement target by the target visual recognition determination unit as the gazing target and the measurement target are sequentially displayed by the target display unit;
wherein the display has a display for a right eye and a display for a left eye; and
wherein the visual field testing unit has a central scotoma possessor testing mode in which the target display unit is controlled so as to cause the gazing target to be displayed by whichever of the display for the right eye or the display for the left eye is opposite an eye being tested.

2. The visual field testing apparatus according to claim 1 **characterized in that**, when in the central scotoma possessor testing mode, the visual field testing unit controls the target display unit so as to cause the gazing target to be displayed at the display for the right eye and so as to cause the measurement target to be displayed at the display for the left eye when testing the left eye of the user, so as to cause the gazing target to be displayed at the display for the left eye and so as to cause the measurement target to be displayed at the display for the right eye when testing the right eye of the user.

3. The visual field testing apparatus according to claim 1 or 2 **characterized in that**, when in the central scotoma possessor testing mode, the visual field testing unit controls the target display unit so as to cause the gazing target to be displayed with increased brightness when the gazing target is displayed by whichever of the display for the right eye or the display for the left eye is opposite the eye being tested.

4. The visual field testing apparatus according to any one of claims 1 through 3 **characterized in that**, when in the central scotoma possessor testing mode, the visual field testing unit controls the target display unit so as to cause the gazing target to be displayed so as to be of increased size when the gazing target is displayed by whichever of the display for the right eye or the display for the left eye is opposite the eye being tested.

5. The visual field testing apparatus according to any one of claims 1 through 4 **characterized in that** the line of sight detection unit, the visual recognition determination unit, the target display unit, and the visual field testing unit employ a polar coordinate system.

6. The visual field testing apparatus according to claim 5 **characterized in that** the visual recognition determination unit determines that visual recognition has occurred when, in the polar coordinate system, a difference between a target angle at the target and a line of sight angle at the line of sight information is not greater than a prescribed angle.
